# EUROPEAN PATENT SPECIFICATION

(11) **EP 2 272 452 B1**
(45) Date of publication and mention of the grant of the patent: **17.04.2013**
(21) Application number: 09009030.9
(22) Date of filing: 10.07.2009
(51) Int. Cl.: A61B 17/88, A61M 25/09

(54) **Guide device for a guide wire pusher**
Führungsvorrichtung für einen Führungsdrahtschieber
Dispositif de guide pour pousseur de guide-fil

(43) Date of publication of application: 12.01.2011
(73) Proprietor: Stryker Trauma GmbH, 24232 Schönkirchen (DE)
(72) Inventor: Prien, Ole, 24145 Kiel (DE); Menzel, Maike, 24149 Kiel (DE)
(74) Representative: Röthinger, Rainer

(56) References cited:
- EP-A2- 0 328 760
- US-A1- 2005 137 601
- US-A1- 2005 177 043

## Description

### Technical Field

The present invention relates to a surgical assembly including a guide device and a guide wire pusher.

### Technical Background

In the field of orthopaedic surgery, it is sometimes necessary to drill into a bone through an area of bone marrow. A guide wire is inserted into the interior of the bone containing bone marrow, and a drill bit having a long, flexible shaft is guided by the guide wire into the desired position.

In order to drill a hole of a desired size, typically several different drill bits of different sizes will be used. After a drill bit has been used to make a hole, the drill bit is removed and another larger drill bit is inserted. Each time the drill bit is withdrawn, there is a risk that the guide wire is withdrawn with the drill bit. Because withdrawing the guide wire causes inconvenience to the patient and operator, and may lengthen the amount of time needed for surgery because the guide wire must be again inserted and positioned for each drill bit used, it is desirable to avoid withdrawing the guide wire each time the drill bit is changed.

The guide wire is typically positioned in an axial recess in a surgical drilling instrument. In order to prevent having to remove the guide wire from its guided position in the bone, a guide wire pusher may be used to push the guide wire in relation to the surgical instrument when the surgical instrument is withdrawn distally over the guide wire such that the guide wire may be maintained in its guided position.

The use of a guide wire and a guide wire pusher provides the advantage that the guide wire will not be withdrawn each time the drill bit is changed, saving the surgeon time and effort, and shortening the overall time in surgery. However, this system may also present the problem that, because both the guide wire and the guide wire pusher have small diameters, it may be difficult to place the two components in concentric engagement such that the guide wire pusher can axially contact the guide wire.

From U.S. Pat. Appl. Publ. 2005/0137601 A1 a guide pin introducer-stylet assembly is known for accessing treatment sites within a spine. The guide pin introducer comprises an introducer handle and an introducer tube. The introducer handle receives at its distal end the introducer tube and has a tubular member lumen which is tapered toward its distal end such that the diameter of the tubular member lumen matches the diameter of the introducer tube. The stylet comprising a stylet rod and a stylet handle is formed such that the stylet is fully received by the guide pin introducer, apart from a small blunt tip and a proximal end which protrude at the distal and proximal end of the guide pin introducer, respectively. The protruding blunt tip is adapted to facilitate an advancement of the guide bin introducer tube to a target site. After reaching the target site, the stylet is removed leaving behind the guide pin introducer with a cavity, through which a guide pin can be guided to the targeted site.

U.S. Pat. Appl. Publ. 2005/0177043 A1 discloses an insertion tool for use in inserting a guide wire into a guide wire lumen of a catheter. The insertion tool comprises a main body having a main lumen extending therethrough wherein the main lumen size is adapted to accommodate the catheter therein. Further, it comprises a funnel-shaped extension connected to and disposed atop the main body, wherein the funnel-shaped extension tapers from the top opening to the bottom opening and is adapted to guide a guide wire which is to be inserted into the guide wire lumen of the catheter.

European Patent Publication 0 328 760 discloses a device for inserting a guide wire into a catheter. A distal portion of the catheter is attached to a head element which is longitudinally divisible such that the head may be opened into two pieces. A funnel-shaped recess is located in the head such that the head element facilitates insertion of the guide wire, because the guide wire is guided by the inner surface of the funnel-shaped section and is forced into the catheter that is held at a proximal end of the head element.

U.S. Pat. Appl. Publ. 2005/0159770 A1 discloses a cone-shaped portion fixed to a proximal end of a guide catheter. The funnel may be collapsed or expanded by adjusting the tension of a wire controlling the size of the cone-shaped portion. The cone-shaped portion may be expanded to facilitate a smooth retrieval of an insertion device.

### Summary

It is an object of the present invention to provide a guide device and a guide wire pusher that facilitate the concentric engagement of a guide wire and the guide wire pusher. It is also an object of the invention to provide a surgical assembly comprising the guide device and the guide wire pusher.

In order to achieve these objects, a guide wire pusher assembly according to claim 1 is provided.

According to an aspect of the invention, the first portion is essentially cylindrical. The first portion may have ridges or recesses provided on the interior surface of the first portion. Frictional resistance may be provided on only one or on more points or sections of the first portion. The internal cross section of the first portion may further be adapted to center the first and second portions relative to the guide wire pusher.

The second portion may essentially be conical. Accordingly, the second portion may have any shape that expands along the length of the guide wire pusher, for instance, the second portion may be funnel-shaped. The inner surface of the second portion may be adapted to allow a guide wire distal end to slide along the inner surface of the second portion, for instance, when axial pressure is applied to the handle of the guide wire pusher.

According to another variant, a proximal end of the guide device provides radial support to the guide wire pusher when the guide device is axially urged against a surface perpendicular to an axial direction of the guide device. The surface may belong to a surgical instrument.

The first portion may further comprise a guide device stopper adapted to engage a guide wire pusher stopper disposed on the guide wire. The guide wire pusher stopper may comprise an area of reduced diameter of the pushing rod. The guide device stopper may comprise an area of reduced diameter adapted to engage the guide wire pusher stopper to prevent the guide device from sliding off the proximal end of the pushing rod. The guide device stopper may be dimensioned to frictionally engage the pushing rod.

The guide wire pusher assembly may further comprise the guide wire disposed in the axial recess of the surgical instrument, for instance, a drill or a surgical screw driver, such that a distal end of the guide wire extends out of a distal end of the surgical instrument. When the guide device is disposed on the guide wire pusher such that the second portion is disposed proximally of the proximal end of the pushing rod, the guide device is adapted to guide the guide wire into concentric engagement with the guide wire pusher by a sliding of the distal end of the guide wire along the second portion of the guide device.

A method of aligning a guide wire and a guide wire pusher comprises the steps of positioning a guide device having a first portion and a second portion on a guide wire pusher such that the second portion of the guide device extends proximally beyond a proximal end of the guide wire pusher and the guide wire pusher extends in frictional contact through at least a section of the first portion, applying axial pressure to the guide wire pusher such that the guide device contacts a distal end of a guide wire disposed in an axial recess in a surgical instrument, further applying axial pressure to the guide wire pusher such that the guide device is urged axially forward, wherein the second portion causes radial forces to be exerted on the contact point between the guide wire and the guide device such that the guide wire is urged into concentric contact with the guide wire pusher, optionally further applying axial pressure to the guide wire pusher such that the guide device contacts a distal surface of the surgical instrument, and further applying axial pressure to the guide wire pusher such that the proximal end of the guide wire pusher moves proximally through the axial recess in the surgical instrument, thereby pushing the guide wire. For instance, the operator may hold the guide wire pusher and may pull the surgical instrument distally such that the surgical instrument is withdrawn from the guide wire by pushing the guide wire relative to the surgical instrument with the guide wire pusher.

### Brief Description of the Drawings

The foregoing and other objects, features, aspects and advantages of the present invention will become more apparent from the following detailed description of the present invention when taken in conjunction with the accompanying drawings.
- Fig. 1: is a cross-sectional view showing an embodiment of a surgical assembly;
- Fig. 2: is a cross sectional view showing an embodiment of a guide device;
- Fig. 3: is another cross sectional view showing the guide device;
- Fig. 4: is another cross-sectional view also showing the guide device; and
- Fig. 5: is another cross-sectional view showing the surgical assembly of Fig. 1.

### Detailed Description

Hereinafter, embodiments of the guide device and the surgical assembly will be described with reference to the accompanying figures. The same reference numerals are used to refer to identical elements. In the following, the term "distal" refers to a direction away from a surgical site, and the term "proximal" refers to a direction toward the surgical site.

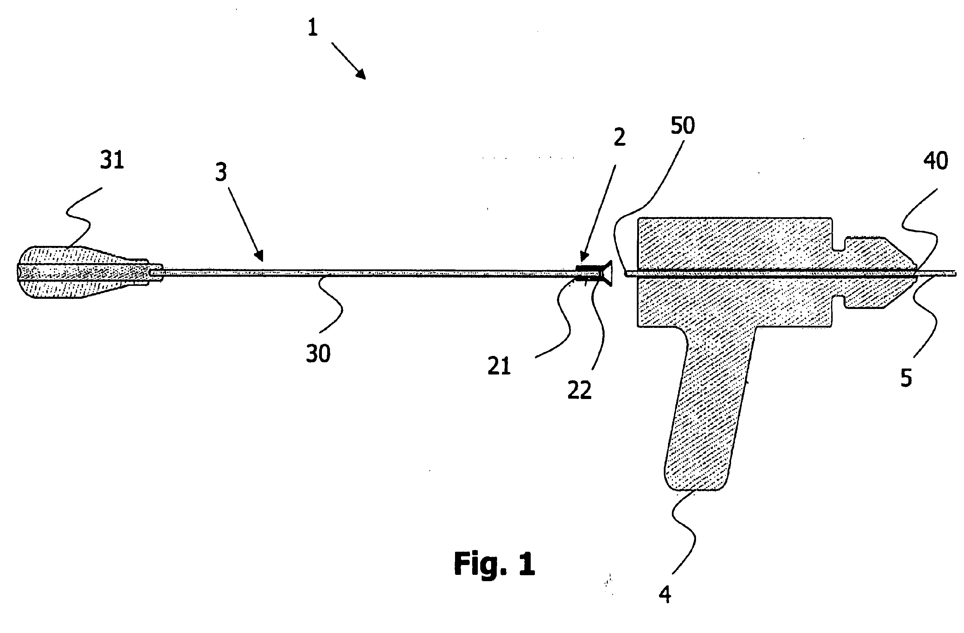

Figure 1 shows a cross sectional view of a surgical assembly 1 comprising a guide device 2 mounted on a guide wire pusher 3. The surgical assembly 1 further comprises a surgical instrument 4 having an axial recess 40. The axial recess 40 extends through the length of the surgical instrument 4. A guide wire 5 is disposed inside the axial recess 40 of the surgical instrument 4. At a proximal end of the guide wire 5, the guide wire 5 is inserted into body tissue (not shown), for instance, into a bone-marrow containing region of the interior of a bone.

A flexible drill bit (not shown) is coupled with the surgical instrument 4 over the guide wire 5 at a proximal end of the surgical instrument 4. The surgical instrument 4 provides the driving force to drive the drill bit for drilling a hole into the bone. The flexible drill bit is guided by the guide wire 5 through the bone marrow region to a desired drilling location on the bone.

The guide wire 5 may have a diameter of approximately 1-8 mm, more preferably 2-5 mm, and even more preferably, approximately 3 mm. The guide wire 5 may be made of metal, plastic, or another suitable, and, preferably, flexible material. The axial recess 40 of the surgical instrument 4 may be sized according to the dimensional requirements of the guide wire 5 such that the guide wire 5 may be accommodated in the axial recess 40.

The guide wire pusher 3 has a pushing rod 30 and a handle 31 disposed on a distal end of the pushing rod 30. The pushing rod 30 may be substantially rigid. Further, the outer surface of the pushing rod 30 may be smooth, or, alternatively, may comprise ridges or recesses. The diameter of the pushing rod 30 may be as described with respect to the guide wire 5.

The pushing rod 30 is also adapted to pass through the distal opening of the axial recess 40 of the surgical instrument 4, and to move axially therethrough. By pushing pushing rod 30 axially through the axial recess 40 of the surgical instrument 4, the pushing rod 30 can prevent the guide wire 5 from being removed from the bone when the surgical instrument 4 is withdrawn. Therefore, the pushing rod 30 can act as a stopper or counter-bearing for the guide wire 5 such that the guide wire 5 may be held in place.

When drilling a hole into bone, a first drill bit is used to drill a starter hole, then successively larger bits are used to increase the size of the drilled hole until the desired diameter is obtained. For example, a size of the drill bits may be increased in diameter by 1 mm for each successive drilling operation.

After each drilling operation, the drill bit must be removed from the drilling site to be exchanged for a drill bit having the next higher diameter. To effect the removal of the drill bit, the surgical instrument 4 is withdrawn distally, and the guide wire pusher 3 pushes the guide wire 5 in relation to the surgical instrument 4 such that the guide wire 5 may remain in position when the surgical instrument 4 extracted. When the surgical instrument 4 and drill bit have been withdrawn from the guide wire 5, the drill bit may be exchanged, and the drill bit and surgical instrument 4 may again be guided into the desired position in the bone by the guide wire 5.

The guide wire 5 may be disposed in a state in which a distal end 50 of the guide wire 5 extends from a distal end of the surgical instrument 4 as shown in Fig. 1. It may be difficult for an operator to cause the guide wire pusher 3 to engage the extending guide wire 5, especially because the guide wire 5 is flexible.

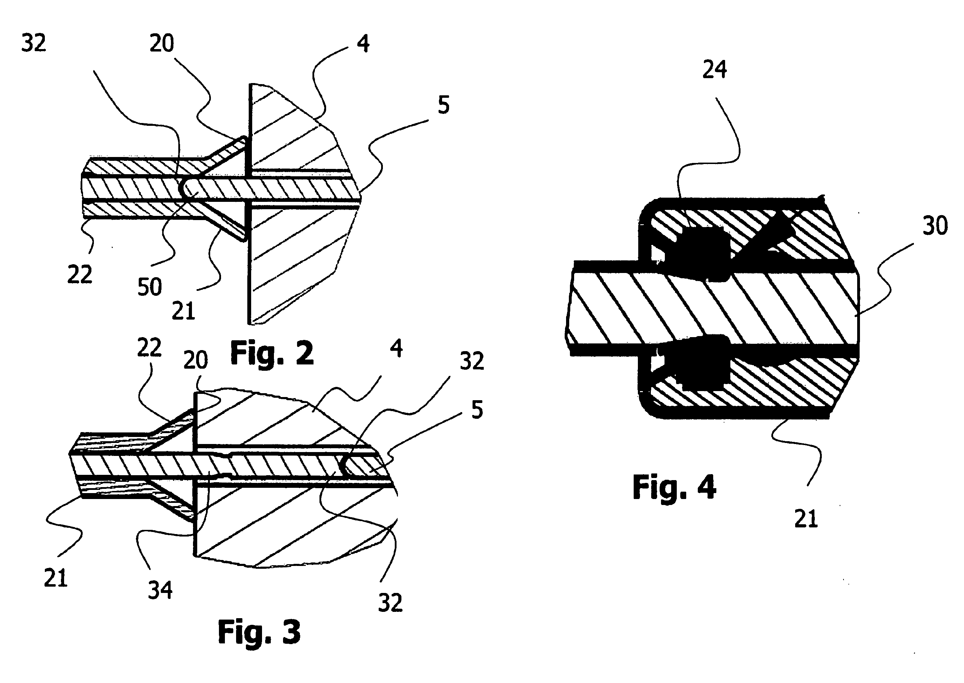

To aid the operator in centring a proximal end 32 of the guide wire pusher 3 with the distal end 50 of the guide wire as shown in Fig. 2, the guide device 2 is initially disposed near the proximal end 32 of the pushing rod 30 as illustrated in Fig. 1. The guide device 2 may be made from metal, plastic, or a combination thereof. Especially when the guide device 2 is made from plastic, it may be detachable from the guide rod 30 for being replaced after each surgical operation.

The guide device 2 comprises a first portion 21 and a second portion 22 that is coaxial with the first portion 21. The first portion 21 has an internal cross section that is adapted to frictionally engage an outer surface of the pushing rod 30. The first portion 21 has a hollow cylindrical shape. Further, the size of the first portion 21 is adapted to the outer surface of the pushing rod 30 such that the first portion 21 and the second portion 22 are axially centered relative to the pushing rod 30.

The second portion 22 has a cross sectional shape that expands proximally from the first portion 21. For instance, the second portion 22 may be conical. An inner surface of the second portion 22 may be smooth. A proximal end of the second portion 22 may have a frictionally resistant surface, such as a rubberized surface.

With reference to Figs. 3 and 4, the guide device 2 has a guide device stopper 24, and the pushing rod 30 has a guide wire pusher stopper 34. The guide wire pusher stopper 34 is adapted to engage the guide device stopper 24 to prevent the guide device 2 from inadvertently sliding off the end 32 of the pushing rod 30.

The guide wire pusher stopper 34 is disposed near the proximal end 32 of the pushing rod 30. The guide wire pusher stopper 34 may comprise an area of reduced diameter. For instance, a portion of the pushing rod 30 may be machined off to form the area of reduced diameter. In another example, the pushing rod 30 may be deformed under pressure such that a deformed portion of the pushing rod 30 has a cross section having an elliptical shape (i.e., having a reduced diameter and an enlarged diameter perpendicular to the portion having the reduced diameter). Further, the guide wire pusher stopper 34 may be a recess, or a protrusion integrally molded with the pushing rod 30.

The guide device stopper 24 is disposed on the first portion 21. The guide device stopper 24 may comprise an area of reduced diameter of the guide device 2. For instance, the guide device stopper 24 may be an area of reduced diameter integrally molded with the first portion 21, or an annular, stepped, or angled member secured to the inner circumference of the first portion 21. The guide device stopper 24 may also comprise a material having a high coefficient of friction, such as rubber or a flexible plastic material, to frictionally engage the pushing rod 30.

The guide device 2 is adapted to move axially along the pushing rod 30. When the guide device 2 moves toward the proximal end 32 of the pushing rod 30, the guide device stopper 24 engages the guide wire pusher stopper 34 to prevent the guide device 2 from inadvertently sliding off the proximal end 32 of the pushing rod 30. When the guide device 2 is realized as a disposable item, the engagement may be releasable so as to permit a replacement of the guide device 2 after each surgical operation. Preferably, the guide device stopper 24 and the guide wire pusher stopper 34 are disposed such that, when the stoppers 24, 34 are engaged, a proximal end of the first portion 21 of the guide device 2 extends proximally beyond the proximal end 32 of the pushing rod 30. Further, the stoppers 24, 34 may preferably be disposed such that, when the stoppers 24, 34 are engaged, the pushing rod 30 provides axial support to the first portion 21 such that the guide device 2 is held substantially concentric with the guide wire pusher 3.

The operation of the above-described surgical assembly 1 will now be described in more detail.

With reference to Fig. 1, in a first position, the distal end 50 of the guide wire 5 extends distally from the surgical instrument 4. An operator holds the guide wire pusher 3 and attempts to bring the proximal end 32 of the pushing rod 30 and the distal end 50 of the guide wire 5 into contact. To aid this operation, the guide device 2 is positioned at the proximal end 32 of the pushing rod 30 as shown in Fig. 1. For instance, as described above, this may be a position at which the stoppers 24, 34 are engaged, thus making the positioning of the guide device 2 simpler for the operator. Further, the guide device 2 is positioned on the pushing rod 30 such that the proximal end 32 of the pushing rod 30 is disposed slightly distally of a proximal end of the first portion 21.

The operator then moves the proximal end 32 of the pushing rod 30 toward the guide wire 5 until the distal end 50 of the guide wire 5 contacts the inner surface of the second portion 22 of the guide device 2.

The operator applies further axial pressure, for example, by pushing the handle 31 of the guide wire pusher 3, to cause the guide device 2 to be moved axially forward such that the distal end 50 of the guide wire 5 is caused to slide along the inner surface of the second portion. To this end, the inner surface of the second portion 22 may be composed of a smooth material, for instance, plastic or metal. The inner conical surface of the second portion 22 causes radial forces to be exerted on a contact point between the guide wire 5 and the guide device 2 in response to an axial force exerted by the operator such that the distal end 32 of the pushing rod 30 is guided toward the distal end 50 of the guide wire 5.

Because the first portion 21 is adapted to provide frictional resistance to the pushing rod 30, the guide device 2 does not move substantially along the pushing rod 30 when the distal end 50 of the guide wire 5 slides along the inner surface of the second portion 22. For example, the frictional resistance could be provided by the guide device stopper 24 engaging the guide wire pusher stopper 34 or other portions of the pushing rod 30. Alternatively, or in addition, the frictional resistance could be caused by a frictional interior surface of the cylindrical first portion 21..

The operator applies further axial pressure until the distal end 50 of the guide wire 5 contacts the proximal end 32 of the pushing rod 30 disposed in the first portion 21, as illustrated with respect to Fig. 2. Because the guide wire 5 may contact the pushing rod 30 inside the first portion 21, the cross section of the first portion 21 may cause the guide wire 5 and the pushing rod 30 to become concentrically engaged. Alternatively, the guide wire 5 and guide wire pusher 3 may also be guided into concentric engagement when the guide wire pusher 3 extends slightly from the first portion 21.

A proximal end 20 of the second portion 22 may contact the distal end of the surgical instrument 4 when the guide wire 5 is brought into concentric engagement with the pushing rod 30. When the proximal end 20 of the second portion 22 contacts the distal end of the surgical device 4, the contacting surface of the proximal end 20 may provide radial support to the guide wire pusher 3 by causing frictional resistance to movement of the second portion 22 in relation to the surgical device 4. Thereby, the operation may be further simplified for the operator.

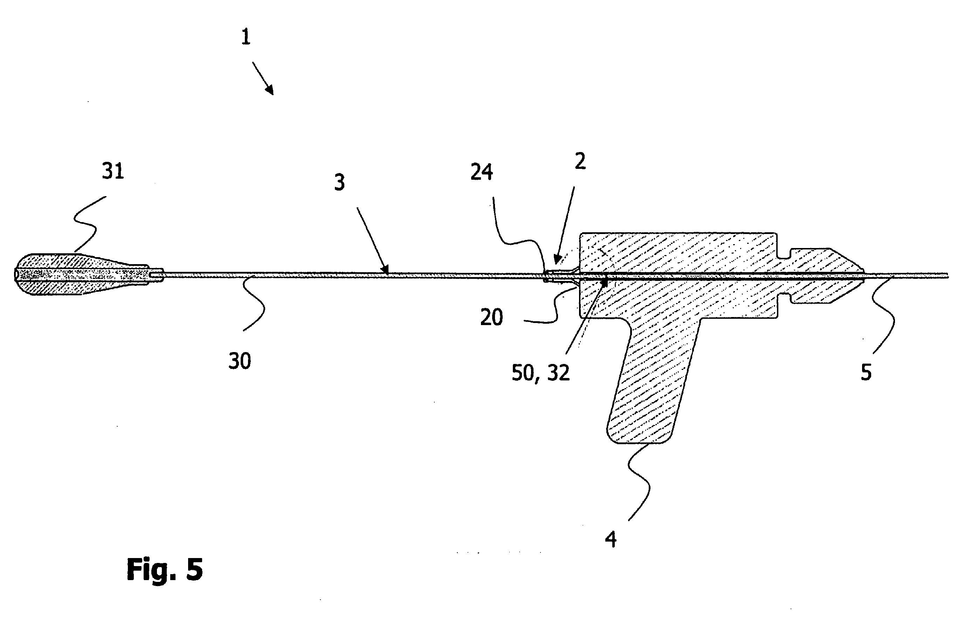

Further, as illustrated with respect to Figs. 3 and 5, the operator may thereafter easily provide a counter-bearing force to the guide wire 5 such that the guide wire 5 may be held in a constant position when the surgical device 4 is withdrawn from the guide wire 5.

As a further example, the proximal end 32 of the pushing rod 30 and the distal end 50 of the guide wire 5 may be further adapted to ease their engagement. For example, one of the two ends 32, 50 may be concave and the other end 32, 50 may be convex, as illustrated with respect to Fig. 3. Alternatively, another type of engaging shape may be provided to the pushing rod 30 and the guide wire 5.

Having described the preferred embodiments of the invention referring to the accompanying drawings, it should be understood that the present invention is not limited to those precise embodiments and various changes and modifications thereof could be made by one skilled in the art without departing from the scope of the invention.

### List of Elements

- 1: surgical assembly
- 2: guide device
- 20: guide device proximal end
- 21: first portion
- 22: second portion
- 24: guide device stopper
- 3: guide wire pusher
- 30: pushing rod
- 31: handle
- 32: pushing rod proximal end
- 34: guide wire pusher stopper
- 4: surgical instrument
- 40: axial recess of surgical instrument
- 5: guide wire
- 50: guide wire distal end

## Claims

1. A guide wire pusher assembly (1) comprising a guide wire pusher (3) having a pushing rod (30) and a guide device (2) disposed on the pushing rod (30), wherein the guide device (2) comprises:
a first portion (21) having an internal cross section adapted to frictionally engage an outer surface of the guide wire pusher (3), and further adapted to allow the guide device (2) to move axially along the guide wire pusher (3); and
a second portion (22) that is coaxial with the first portion (21) and has an internal cross section that expands radially along the length of the second portion (22), the second portion (22) being adapted to center the guide wire pusher (3) relative to a guide wire (5) that is disposed in a recess (40) extending through a length of a surgical instrument (4), wherein the proximal end (32) of the guide wire pusher (3) can move proximally through the recess (40) in the surgical instrument (4), thereby pushing the guide wire (5).

2. The guide wire pusher assembly of claim 1, wherein the first portion (21) is cylindrical.

3. The guide wire pusher assembly of any preceding claim, wherein the second portion (22) is conical.

4. The guide wire pusher assembly of any preceding claim, wherein the internal cross section of the first portion (21) is further adapted to center the first and second portions (21, 22) relative to the guide wire pusher (3).

5. The guide wire pusher assembly of any preceding claim, wherein an inner surface of the second portion (22) is adapted to allow a guide wire distal end (50) to slide along the inner surface of the second portion (22) toward the first portion (21).

6. The guide wire pusher assembly of any preceding claim, wherein a proximal end (20) of the guide device (2) provides radial support to the guide wire pusher (3) when the guide device (2) is axially urged against a surface perpendicular to an axial direction of the guide device (2).

7. The guide wire pusher assembly of any preceding claim, wherein the first portion (21) further comprises a guide device stopper (24) adapted to engage a guide wire pusher stopper (34) disposed on the guide wire pusher (3).

8. The guide wire pusher assembly of any preceding claim, wherein the guide wire pusher (3) further comprises a handle (31) at a distal end thereof.

9. The guide wire pusher assembly of any preceding claim, wherein the pushing rod (30) is substantially rigid.

10. The guide wire pusher assembly of claim 7, wherein the guide wire pusher stopper (34) comprises an area of reduced diameter of the pushing rod (30).

11. The guide wire pusher assembly of claim 10, wherein the guide device stopper (24) comprises an area of reduced diameter adapted to engage the guide wire pusher stopper (34) to prevent the guide device (2) from sliding off a proximal end (32) of the pushing rod (30).

12. The guide wire pusher assembly of any preceding claim, further comprising the guide wire (5) disposed in the recess (40) of the surgical instrument (4) such that a distal end (50) of the guide wire (5) extends out of a distal side of the surgical instrument (4),
wherein, when the guide device (2) is disposed on the guide wire pusher (3) such that the second portion (22) is disposed proximally of the proximal end (32) of the pushing rod (30), the guide device (2) is adapted to guide the guide wire pusher (3) into concentric engagement with the guide wire (5) by a sliding of the distal end (50) of the guide wire (5) along the second portion (22) of the guide device (2).

13. The guide wire pusher assembly of claim 12, wherein the guide device (2) is adapted to allow the guide wire (5) to slide along the second portion (22) of the guide device (2) when axial pressure is applied to the handle (31) of the guide wire pusher (3).

14. The guide wire pusher assembly of claim 12, further comprising the surgical instrument (4), wherein the surgical instrument (4) is a surgical drill.

## Patentansprüche

1. Führungsdrahtschieber-Anordnung (1), umfassend einen Führungsdrahtschieber (3) mit einer Schubstange (30) und einer auf der Schubstange (30) angeordnete Führungsvorrichtung (2),
wobei die Führungsvorrichtung (2) umfasst:
einen ersten Abschnitt (21) mit einem Innenquerschnitt, der dazu ausgebildet ist, reibschlüssig an einer Außenfläche des Führungsdrahtschiebers (3) anzugreifen, und ferner dazu ausgebildet ist, der Führungsvorrichtung (2) zu ermöglichen, sich axial entlang des Führungsdrahtschiebers (3) zu bewegen; und
einen zweiten Abschnitt (22), der koaxial zum ersten Abschnitt (21) ist und einen Innenquerschnitt aufweist, der sich radial entlang der Länge des zweiten Abschnitts (22) aufweitet, wobei der zweite Abschnitt (22) dazu ausgebildet ist, den Führungsdrahtschieber (3) relativ zu einem Führungsdraht (5) zu zentrieren, der in einer Ausnehmung (40) angeordnet ist, die sich über eine Länge eines chirurgischen Instruments (4) erstreckt, wobei sich das proximale Ende (32) des Führungsdrahtschiebers (3) proximal durch die Ausnehmung (40) im chirurgischen Instrument (4) bewegen kann und dadurch den Führungsdraht (5) schiebt.

2. Führungsdrahtschieber-Anordnung nach Anspruch 1, wobei der erste Abschnitt (21) zylindrisch ist.

3. Führungsdrahtschieber-Anordnung nach einem vorhergehenden Anspruch, wobei der zweite Abschnitt (22) konisch ist.

4. Führungsdrahtschieber-Anordnung nach einem vorhergehenden Anspruch, wobei der Innenquerschnitt des ersten Abschnitts (21) ferner dazu ausgebildet ist, den ersten und zweiten Abschnitt (21, 22) relativ zu dem Führungsdrahtschieber (3) zu zentrieren.

5. Führungsdrahtschieber-Anordnung nach einem vorhergehenden Anspruch, wobei eine Innenfläche des zweiten Abschnitts (22) dazu ausgebildet ist, einem distalen Ende (50) des Führungsdrahtes zu ermöglichen, entlang der Innenfläche des zweiten Abschnitts (22) in Richtung des ersten Abschnitts (21) zu gleiten.

6. Führungsdrahtschieber-Anordnung nach einem vorhergehenden Anspruch, wobei ein proximales Ende (20) der Führungsvorrichtung (2) dem Führungsdrahtschieber (3) eine radiale Abstützung bereitstellt, wenn die Führungsvorrichtung (2) axial gegen eine zu einer axialen Richtung der Führungsvorrichtung (2) senkrechten Fläche gedrängt wird.

7. Führungsdrahtschieber-Anordnung nach einem vorhergehenden Anspruch, wobei der erste Abschnitt (21) ferner einen Führungsvorrichtungsstopper (24) aufweist, der dazu ausgebildet ist, an einem auf dem Führungsdrahtschieber (3) angeordneten Führungsdrahtschieberstopper (34) anzugreifen.

8. Führungsdrahtschieber-Anordnung nach einem vorhergehenden Anspruch, wobei der Führungsdrahtschieber (3) ferner an seinem distalen Ende einen Griff (31) aufweist.

9. Führungsdrahtschieber-Anordnung nach einem vorhergehenden Anspruch, wobei die Schubstange (30) im Wesentlichen starr ist.

10. Führungsdrahtschieber-Anordnung nach Anspruch 7, wobei der Führungsdrahtschieberstopper (34) einen Bereich mit verringertem Durchmesser der Schubstange (30) aufweist.

11. Führungsdrahtschieber-Anordnung nach Anspruch 10, wobei der Führungsvorrichtungsstopper (24) einen Bereich mit verringertem Durchmesser aufweist, der dazu ausgebildet ist, an dem Führungsdrahtschieberstopper (34) anzugreifen, um die Führungsvorrichtung (2) am Heruntergleiten von einem proximalen Ende (32) der Schubstange (30) zu hindern.

12. Führungsdrahtschieber-Anordnung nach einem vorhergehenden Anspruch, ferner umfassend den Führungsdraht (5), der in der Ausnehmung (40) des chirurgischen Instruments (4) so angeordnet ist, dass ein distales Ende (50) des Führungsdrahtes (5) an einer distalen Seite des chirurgischen Instruments (4) herausragt,
wobei, wenn die Führungsvorrichtung (2) auf dem Führungsdrahtschieber (3) so angeordnet ist, dass der zweite Abschnitt (22) proximal zum proximalen Ende (32) der Schubstange (30) angeordnet ist, die Führungsvorrichtung (2) dazu ausgebildet ist, den Führungsdrahtschieber (3) durch ein Gleiten des distalen Endes (50) des Führungsdrahtes (5) entlang des zweiten Abschnitts (22) der Führungsvorrichtung (2) in einen konzentrischen Eingriff mit dem Führungsdraht (5) zu führen.

13. Führungsdrahtschieber-Anordnung nach Anspruch 12, wobei die Führungsvorrichtung (2) dazu ausgebildet ist, dem Führungsdraht (5) zu ermöglichen, entlang des zweiten Abschnitts (22) der Führungsvorrichtung (2) zu gleiten, wenn axialer Druck auf den Griff (31) des Führungsdrahtschiebers (3) aufgebracht wird.

14. Führungsdrahtschieber-Anordnung nach Anspruch 12, ferner umfassend das chirurgische Instrument (4), wobei das chirurgische Instrument (4) ein chirurgischer Bohrer ist.

## Revendications

1. Ensemble (1) de pousseur de fil-guide comprenant un pousseur de fil-guide (3) comportant une tige de poussée (30) et un dispositif de guidage (2) disposé sur la tige de poussée (30), dans lequel le dispositif de guidage (2) comprend :
une première partie (21) présentant une section transversale interne adaptée pour se mettre en prise par frottement avec une surface externe du pousseur de fil-guide (3), et en outre adaptée pour permettre au dispositif du guidage (2) de se déplacer axialement le long du pousseur de fil-guide (3) ; et
une deuxième partie (22) qui est coaxiale à la première partie (21) et présente une section transversale interne qui s'étend radialement le long de la longueur de la deuxième partie (22), la deuxième partie (22) étant adaptée pour centrer le pousseur de fil-guide (3) par rapport au fil-guide (5) et qui est disposée dans un évidement (40) s'étendant à travers une longueur d'un instrument chirurgical (4), dans lequel l'extrémité proximale (32) du pousseur de fil-guide (3) peut se déplacer de manière proximale à travers l'évidement (40) dans l'instrument chirurgical (4), d'où il résulte que le fil-guide (5) est poussé.

2. Ensemble de pousseur de fil-guide selon la revendication 1, dans lequel la première partie (21) est cylindrique.

3. Ensemble de pousseur de fil-guide selon l'une quelconque des revendications précédentes, dans lequel la deuxième partie (22) est conique.

4. Ensemble de pousseur de fil-guide selon l'une quelconque des revendications précédentes, dans lequel la section transversale interne de la première partie (21) est en outre adaptée pour centrer les première et deuxième parties (21, 22) par rapport au pousseur de fil-guide (3).

5. Ensemble de pousseur de fil-guide selon l'une quelconque des revendications précédentes, dans lequel une surface interne de la deuxième partie (22) est adaptée pour permettre qu'une extrémité distale du fil-guide (50) glisse le long de la surface interne de la deuxième partie (22) vers la première partie (21).

6. Ensemble de pousseur de fil-guide selon l'une quelconque des revendications précédentes, dans lequel une extrémité proximale (20) du dispositif de guidage (2) fournit un support radial au pousseur de fil-guide (3) lorsque le dispositif du guidage (2) est sollicité axialement contre une surface perpendiculaire à une direction axiale du dispositif de guidage (2).

7. Ensemble de pousseur de fil-guide selon l'une quelconque des revendications précédentes, dans lequel la première partie (21) comprend en outre une butée de dispositif de guidage (24) adaptée pour se mettre en prise avec une butée de pousseur de fil-guide (34) disposée sur le pousseur de fil-guide (3).

8. Ensemble de pousseur de fil-guide selon l'une quelconque des revendications précédentes, dans lequel le pousseur de fil-guide (3) comprend en outre une poignée (31) au niveau d'une extrémité distale de celui.

9. Ensemble de pousseur de fil-guide selon l'une quelconque des revendications précédentes, dans lequel la tige de poussée (30) est substantiellement rigide.

10. Ensemble de pousseur de fil-guide selon la revendication 7, dans lequel la butée du pousseur de fil-guide (34) comprend une zone à diamètre réduit de la tige de poussée (30).

11. Ensemble de pousseur de fil-guide selon la revendication d'ici, dans lequel la butée du dispositif de guidage (24) comprend une zone à diamètre réduit adaptée pour se mettre en prise avec la butée du pousseur de fil-guide (34) pour empêcher que le dispositif de guidage (2) glisse d'une extrémité proximale (32) de la tige de poussée (30).

12. Ensemble de pousseur de fil-guide selon l'une quelconque des revendications précédentes, comprenant en outre le fil-guide (5) disposé dans l'évidement (40) de l'instrument chirurgical (4) d'une manière telle qu'une extrémité distale (50) du fil-guide (5) s'étend hors d'un côté distal de l'instrument chirurgical (4),
dans lequel, lorsque le dispositif de guidage (2) est disposé sur le pousseur de fil-guide (3) d'une manière telle que la deuxième partie (22) est disposée de façon proximale par rapport à l'extrémité proximale (32) de la tige de poussée (30), le dispositif de guidage (2) est adapté pour guider le pousseur de fil-guide (roi) en prise concentrique avec le fil-guide (5) par un glissement de l'extrémité distale (50) du fil-guide (5) le long de la deuxième partie (22) du dispositif du guidage (2).

13. Ensemble de pousseur de fil-guide selon la revendication 12, dans lequel le dispositif de guidage (2) est adapté pour permettre au fil-guide (5) de glisser le long de la deuxième partie (22) du dispositif de guidage (2) lorsqu'une pression axiale est appliquée à la poignée (31) du pousseur de fil-guide (3).

14. Ensemble de pousseur de fil-guide selon la revendication 12, comprenant en outre l'instrument chirurgical (4), dans lequel l'instrument chirurgical (4) est un foret chirurgical.
